# EUROPÄISCHE PATENTANMELDUNG

(11) **EP 2 924 418 A1**
(43) Veröffentlichungstag der Anmeldung: **30.09.2015**
(21) Anmeldenummer: 15155093.6
(22) Anmeldetag: 13.02.2015
(51) Int. Cl.: G01N 21/82, G01N 33/28

(54) **Verfahren zur Ermittlung des Gesamteisengehalts in einer Probe eines flüssigen Schmieröls**

(30) Priorität: 25.02.2014 EP 14156620
(71) Anmelder: CM Technologies GmbH, 35337 Elmshorn (DE)
(72) Erfinder: Jeske, Andreas, 25335 Elmshorn (DE); Winkler, Matthias, 25337 Elmshorn (DE)
(74) Vertreter: Meyer, Ludgerus

(57) **Zusammenfassung**

Die Erfindung betrifft ein Verfahren zur Ermittlung des Gesamteisengehalts in einer Probe eines flüssigen Schmieröls, insbesondere einer Schiffsmaschine, mittels folgender Schritte:
a) Aus dem Schmieröl wird eine Probe entnommen,
b) Die Probe, eine eisenionenbildende Säure und eine Lösung zweier Eisenkomplexbildner werden einem Mischbehälter zugeführt und durch Schütteln vermischt,
c) Der Mischbehälter wird in ein Ultraschallbad eingebracht und bei erhöhter Temperatur wenigstens solange einem Ultraschallfeld unterzogen, bis eine Phasentrennung in der Mischung mit einer schwereren Phase, welche Eisenionen enthält, und einer leichteren Phase, welche Ölreste enthält, erfolgt ist,
d) Der Eisengehalt der Probe wird über die Feststellung der Trübung, der Chrominanz oder der Luminanz einer Farbbildung in dem Reaktionsgefäß ausgewertet.

## Beschreibung

Die Erfindung betrifft ein Verfahren zur Ermittlung des Gesamteisengehalts in einer Probe eines Schmieröls, insbesondere einer Schiffsmaschine.

Die in Handelsschiffen, insbesondere Containerschiffen, Tankern usw. verwendeten Schiffsmaschinen sind in der Regel langsam laufende Motoren mit fünf und mehr Zylindern, wobei die Motoren eine Höhe von mehr als 10 Metern erreichen können. Die gewünschte hohe Lebensdauer von über 20 Jahren erfordert es, dass der Betriebszustand solcher Motoren ständig überwacht wird.

Bei Großdieselmotoren für einen derartigen Einsatz erfolgt die Schmierung der Kolbenringe zu den Laufbuchsen in der Regel getrennt von der Schmierung des Kurbeltriebes. Dabei erfolgt die Schmierung zwischen den Kolbenringen und Laufbuchsen häufig als Verlustschmierung, wobei verbranntes Schmieröl bei jedem Kolbenhub durch den untersten Kolbenring abgestreift und entsorgt wird. Große Containerschiffe können bis zu einer Tonne Schmieröl pro Tag "verbrauchen". Es besteht daher das Bestreben, den Schmierölverbrauch möglichst weit zu reduzieren, ohne dass gleichzeitig die Gefahr vergrößert wird, eine Unterschmierung hervorzurufen, was zu großen Schäden am Motor führen kann.

Der Verschleiß eines Großdieselmotors tritt einerseits als mechanischer Abrieb auf, andererseits jedoch auch durch Säurekorrosion aufgrund bestimmter Kraftstoffeigenschaften, wie z. B. dem Schwefelgehalt. Korrosion tritt verstärkt dann auf, wenn ein Motor nicht bei optimalen Bedingungen betrieben wird, sondern der Motor beispielsweise bei langsamer Fahrt mit niedrigerer Temperatur arbeitet.

Im Schmieröl eines Motors finden sich daher nicht nur Abriebeisenpartikel, sondern z. B. auch korrodierte Eisenteilchen in Form von Eisenoxyd oder Eisensulfat. Um eine lange Lebensdauer des Motors zu gewährleisten, ist es notwendig, ständig den genauen Eisenanteil, sowohl des Abriebeisens als auch des korrodierten Eisens, zu überwachen.

Bei einer Verlustschmierung ist der Eisenanteil auch ein Gradmesser für den momentanen Verschleißzustand von Zylindern und Kolbenringen.

Zu Bestimmung des Alterungszustandes eines Schmieröls sind verschiedene Messverfahren bekannt, die unterschiedliche Eigenschaften des Schmieröls erfassen. Dazu gehören neben dem Eisengehalt die Säurezahl, der Additivgehalt, die Basenzahl, die Viskosität und die Leitfähigkeit.

Aus der DE 197 06 486 B4 ist eine Sensoreinrichtung zur Bestimmung des Alterungszustandes flüssiger Öle bekannt, welche zwei Interdigitalwandler zur Erzeugung einer elektroakustischen Welle verwendet, wobei zur Bewertung des Öls die ermittelte Resonanzfrequenz oder die Dämpfung der elektroakustischen Welle zwischen den Interdigitalwandlern ausgewertet wird, insbesondere indem die komplexe elektrische Impedanz mittels einer Auswertungsschaltung über eine Auswertung einer zweiten Resonanzfrequenz ermittelt wird.

Es ist auch bekannt, die magnetischen oder Leiteigenschaften einer Testflüssigkeit dadurch auszuwerten, dass magnetisierbare Partikel durch Änderung der Impedanz in einem elektrischen Feld gemessen werden, siehe US 6,051,970.

Das vorstehende Verfahren ist jedoch nur dafür geeignet, magnetisierbare Eisenteilchen in einem Öl festzustellen oder andere magnetfeldabhängige Parameter zu ermitteln.

Aus der US 2006/270050 A1 ist ein Verfahren zur Bestimmung des Eisengehalts in einem Schmieröl bekannt, bei dem eine Probe des Schmieröls einem aktiven Lösungsmittel mit einem pH-Wert zwischen 2 und 4 hinzugefügt wird, das ein apolares Lösungsmittel, ein polares Lösungsmittel, eine Säure, Wasser, einen Eisenkomplexbildner und ein Reduktionsmittel enthält. Es erfolgt eine intensive Mischung, solange bis die Probe vollständig aufgelöst ist. Die Mischung soll sich dann in eine obere und eine untere Schicht trennen, wobei die untere Schicht einen Eisenkomplex enthält, der ausgefiltert und zur photometrischen Absorptionsmessung im sichtbaren Bereich bereitgestellt wird. Daraus lässt sich der ppm-Wert an Eisen in der Probe ermitteln.

Es ist Aufgabe der Erfindung, ein Verfahren zur Ermittlung des Gesamteisengehalts eines Schmieröls anzugeben, mit dem der momentane Verschleißzustand eines Motors und insbesondere dessen Änderung schnell und einfach ermittelt werden kann.

Diese Aufgabe wird durch die in Anspruch 1 angegebene Erfindung gelöst. Vorteilhafte Weiterbildungen der Erfindung sind in den Unteransprüchen angegeben.

Das erfindungsgemäße Verfahren zur Ermittlung des Gesamteisengehaltes in einer Probe eines flüssigen Schmieröls besteht insbesondere darin, zunächst eine Probe eines Schmieröls in einem Mischbehälter mit einer Eisenionen bildenden Säure und einem Eisenkomplexbildner durch starkes Schütteln des Mischbehälters zu vermischen. Der Mischbehälter wird dann in ein Ultraschallbad eingebracht. Bei erhöhter Temperatur wird die Mischung mindestens so lange einem Ultraschallfeld unterzogen, bis eine Phasentrennung in der Mischung in zwei Phasen mit unterschiedlichem spezifischem Gewicht erfolgt, wobei die schwerere Phase, welche Eisenionen enthält, und die leichtere Phase, welche Ölreste enthält, durch eine Phasentrennebene voneinander getrennt sind. Die schwerere Phase, d.h. die Flüssigkeitsphase, welche ein höheres spezifisches Gewicht aufweist, wird dann auf Trübung oder Färbung der Flüssigkeit ausgewertet, welche als Maß für den Gesamteisengehalt in der Probe dient.

Es wird bevorzugt, als Eisenionen bildende Säure verdünnte Salpetersäure zu verwenden, insbesondere, weil diese in der Handhabung insbesondere im rauen Schiffsbetrieb relativ ungefährlich ist, obgleich auch andere Säuren, insbesondere Salzsäure, verwendbar sind. Vorzugsweise wird die Eisenionen bildende Säure, die als Salpetersäure eine 0,01 - 1 molare Lösung in H₂O sein kann, zunächst in einen Mischbehälter eingegeben, dem dann die Probe in einem 0,01 : 1 bis 0,5 : 1 Verhältnis von Probe zu Säure zugeführt wird.

Der Mischbehälter ist vorzugsweise ein zylindrisches Röhrchen, in die die Eisenionen bildende Säure, der Eisenkomplexbildner und die Probe eingebracht werden, wobei zur Erleichterung der Mischung ein Teilvolumenraum des Mischbehälters mit Luft gefüllt bleibt.

Das Einbringen des Mischbehälters in einen Ulltraschallerzeuger erfolgt bei einem zylindrischen Behälter mit gegenüber dem Durchmesser großer Höhe vorzugsweise in geneigter Stellung des Mischbehälters, derart, dass die sich bei der Ultraschallbehandlung ergebende Phasentrennung eine möglichst große Trennebene bzw. Trennfläche hervorruft. Dieses erleichtert sowohl die Durchmischung der Eisenionen bildenden Säure mit der Ölprobe als auch die anschließende Ausbildung der Phasentrennebene.

Alternativ kann ein Mischbehälter verwendet werden, dessen Höhe kleiner als der Durchmesser gewählt ist, wodurch keine Neigung des Mischbehälters erforderlich ist.

Als Mischbehälter sollte ein Behälter mit harter Oberfläche benutzt werden. Vorzugsweise wird ein Mischbehälter aus Glas verwendet.

Im Ultraschallbad erfolgt die Behandlung insbesondere mit Ultraschall mit einer Leistung von > 20 W, einer Ultraschallfrequenz von > 30 kHz und einer Zeitdauer von wenigstens 5 min bei einer Temperatur des Ultraschallbades von > 30°C. In einem bevorzugten Ausführungsbeispiel wird eine Leistung des Ultraschallerzeugers von 30 bis 60 W, eine Ultraschallfrequenz von 35 bis 45 kHz und eine Zeitdauer von 10 bis 60 min bei einer Temperatur des Ultraschallbades von 30 - 50°C gewählt. Diese Parameter können in der praktischen Umsetzung anhand der erzielten Ergebnisse weiter optimiert werden.

Durch den Einsatz von Ultraschall werden die Flüssigkeiten im Mischbehälter, nämlich die Ölphase mit abrasiven und korrosiven Eisenbestandteilen und die wässrige HNO3-Phase, in kleinste Schwingungen versetzt, wodurch sich folgende Vorteile ergeben:
a) Für korrosives Eisen: Durch die anfängliche Zufuhr mechanischer Energie (Schütteln) werden temporär zwei Phasen (HNO₃ und Öl) die sich unter Normalbedingungen nicht stabil durchmengen lassen, vermischt. Dadurch wird erreicht, dass möglichst viel Säure mit dem im Öl vorhandenen Eisen in Kontakt kommt. Danach wird der Mischbehälter in das Ultraschallbad eingebracht. Hier wird die natürliche Trennung (Entmischung) der beiden Phasen durch Einwirkung von Temperatur und Ultraschallenergie beschleunigt. Danach erfolgt durch die erhöhte Temperatur und die Ultraschallenergie eine Konvektion, die ständig eine Veränderung der Molekülzusammensetzung an der Phasentrennebene bewirkt. Dieser Prozess fördert den immer weiteren Übertrag von ionisiertem Eisen in die wässrige HNO₃-Phase.
b) Für abrasives Eisen: Kleinste abrasive Eisenteilchen im zweistelligen Nanometerbereich sinken in Stoffen wie Ölen mit hoher Viskosität nicht zeitnah selbsttätig ab, trotz höherer Dichte der Eisenteilchen gegenüber dem Öl, wie das in dünneren Medien, wie zum Beispiel Wasser, oder bei größeren Teilchen der Fall wäre. Mittels Ultraschall werden diese nun "herausgeschüttelt" und "wandern" in die untere Phase. Die Temperatur erhöht die Brownsche Molekularbewegung der Teilchen, die in diesem Zusammenhang die Viskosität der Ölphase herabsetzt, wodurch dieser Effekt weiter unterstützt wird.

Der Eisenkomplexbildner kann sowohl ein Eisen-II- als auch ein Eisen-III-Komplexbildner oder auch eine Mischung davon sein. Als Eisen-II-Komplexbildner wird insbesondere Kaliumhexazyanidoferrat-III und als Eisen-III-Komplexbildner Kaliumhexazyanodidoferrat-II verwendet. Kaliumhexazyanidoferrat-II ist auch als gelbes Blutlaugensalz bekannt, während Kaliumhexazyanodidoferrat-III als rotes Blutlaugensalz bekannt ist, welche beide häufig zur Herstellung von Blaupigmenten und Blaudrucken verwendet werden. Bei der Vermischung des gelben bzw. roten Blutlaugensalzes mit Eisenionen entsteht das sogenannte Berliner Blau, das sich durch Blaufärbung der erhaltenen Lösung kennzeichnet. Der Grad der Verfärbung in Form einer Trübung, der Änderung der Chrominanz oder der Luminanz ist ein Maß für die ermittelte Eisenionenmenge, wobei vorzugsweise die Feststellung der Trübung im Infrarotbereich bei 800 - 900 nm, weiter vorzugsweise bei 850 nm erfolgt.

Die Auswertung kann durch Augenschein vorgenommen werden, jedoch wird es bevorzugt, die Auswertung durch ein Trübungs- oder Färbungsmessgerät bekannter Art vorzunehmen, um reproduzierbare Ergebnisse dokumentieren zu können.

Mit dem vorstehenden Verfahren ist es möglich, den Gesamteisengehalt in einer Probe eines Öls, insbesondere Schmieröls einer Schiffsmaschine, zu bestimmen, und damit den Verschleißzustand des Öls und/oder des Motors ermitteln zu können. Die erfindungsgemäßen Verfahrensschritte können unmittelbar an Bord eines Schiffes durchgeführt werden, ohne dass dafür eine komplexe Labortechnik vorhanden sein muss.

Die Erfindung wird nachstehend anhand eines Beispiels näher erläutert.

In einem Beispiel wurde als Probenbehälter ein 10 ml Glasspritzenbehälter mit einem Druck- oder Zugkolben verwendet, in den durch Aufziehen des Kolbens 9 ml einer 0,1-molaren Lösung von Salpetersäure in H₂O sowie eine 1 ml Lösung aus gelbem und rotem Blutlaugensalz in 140 mg/l gefiltertem Wasser aufgezogen wurde. Es wurden ferner eine repräsentative Probe von 1 ml Schmieröl aus einer Schiffsmaschine sowie weitere 2 ml Luft in die Spritze aufgezogen. Es folgte eine initiale Vermischung der Salpetersäure mit der Probe durch händisches Schütteln über 60 sec oder etwa 30-maliges Hin- und Herschütteln. Dann wurde die Spritze in ein Ultraschallbad eingebracht, das eine Leistung von 50 W bei einer Ultraschallfrequenz von 42 kHz aufwies. Die Temperatur des Ultraschallbades betrug 40°C. Der Mischbehälter wurde für 15 min in dem Ultraschallbad behandelt, wobei der Mischbehälter (die Spritze) schräg im Ultraschallbad gelagert wurde, so dass in dem Mischbehälter eine möglichst große Fläche der Phasentrennebene zwischen der leichteren und der schwereren Phase erreicht werden konnte. Nach Beendigung der Ultraschallbehandlung wurde die Spritze aufrecht aus dem Ultraschallbad entnommen und in die Messzelle eines fotoelektrischen Messgeräts eingebracht, um die Trübung bzw. Färbung der erhaltenen Lösung im Infrarotbereich bei 850 nm festzustellen. Durchgeführte Messreihen mit einem Frischöl, einem stark mit Eisen versetztem Schmieröl und Ölen mit verschiedenen Abstufungen des Gesamteisengehaltes führten zu wiederholbaren Ergebnissen, die ein Maß für den Gesamteisengehalt einer Probe ergaben.

In einem weiteren Versuch wurde festgestellt, dass die Verweildauer der Probenmischung im Ultraschallbad 10 min nicht unterschreiten sollte, da bei geringerer Zeitdauer keine ausreichende Trennung zwischen der Eisenionen enthaltenden Phase und Ölresten erhältlich war. Grundsätzlich war es von Vorteil, die Temperatur der Ultraschallbehandlung zu erhöhen, jedoch ohne eine Temperatur von 50°C zu überschreiten.

## Patentansprüche

1. Verfahren zur Ermittlung des Gesamteisengehalts in einer Probe eines flüssigen Schmieröls, insbesondere einer Schiffsmaschine, mittels folgender Schritte:
a) Aus dem Schmieröl wird eine Probe entnommen,
b) Die Probe, eine eisenionenbildende Säure und eine Lösung eines Eisenkomplexbildners werden einem Mischbehälter zugeführt und durch Schütteln vermischt,
c) Der Mischbehälter wird in ein Ultraschallbad eingebracht und bei erhöhter Temperatur wenigstens solange einem Ultraschallfeld unterzogen, bis eine Phasentrennung in der Mischung mit einer schwereren Phase, welche Eisenionen enthält, und einer leichteren Phase, welche Ölreste enthält, erfolgt ist,
d) Der Eisengehalt der Probe wird über die Feststellung der Trübung der schwereren Phase in dem Reaktionsgefäß ausgewertet.

2. Verfahren nach Anspruch 1, **dadurch gekennzeichnet, dass** die eisenionenbildende Säure verdünnte Salpetersäure (HNO₃₎ ist.

3. Verfahren nach Anspruch 1 oder 2, **dadurch gekennzeichnet, dass** die Probe und die eisenionenbildende Säure in einem Verhältnis 0,01 : 1 bis 0,5 : 1 Volumenanteile dem Mischbehälter zugeführt werden.

4. Verfahren nach Anspruch 3, **dadurch gekennzeichnet, dass** die Volumina von Probe und eisenionenbildender Säure 0,1 : 1 betragen.

5. Verfahren nach Anspruch 4, **dadurch gekennzeichnet, dass** die Volumina jeweils 1 - 40 ml betragen.

6. Verfahren nach Anspruch 2, **dadurch gekennzeichnet, dass** die Salpetersäure eine 0,01 - 1 molare Lösung in H₂O ist.

7. Verfahren nach einem der Ansprüche 1 - 5, **dadurch gekennzeichnet, dass** nach dem Einbringen von Probe und Salpetersäure in einen im wesentlichen zylindrischen Mischbehälter der Mischbehälter so geneigt ist, dass die Fläche der Phasentrennung maximiert ist.

8. Verfahren nach Anspruch 1, **dadurch gekennzeichnet, dass** die Behandlung im Ultraschallbad mit einem Ultraschallerzeuger mit einer Leistung von > 20 Watt, einer Ultraschallfrequenz von > 30 kHz und einer Zeitdauer von wenigstens 5 min bei einer Temperatur des Ultraschallbades von > 30 °C erfolgt.

9. Verfahren nach Anspruch 8, **dadurch gekennzeichnet, dass** die Behandlung im Ultraschallbad mit einem Ultraschallerzeuger mit einer Leistung von 30 - 60 Watt, einer Ultraschallfrequenz von 35 - 45 kHz und einer Zeitdauer von 10 - 60 min bei einer Temperatur des Ultraschallbades von 30 - 50 °C erfolgt.

10. Verfahren nach Anspruch 1, **dadurch gekennzeichnet, dass** der Eisenkomplexbildner ein Eisen-II- und Eisen-III-Komplexbildner ist.

11. Verfahren nach Anspruch 10, **dadurch gekennzeichnet, dass** der Eisen-III-Komplexbildner Kaliumhexacyanidoferrat (II) K₄[Fe(CN)₆] ist.

12. Verfahren nach Anspruch 10, **dadurch gekennzeichnet, dass** der Eisen-II-Komplexbildner Kaliumhexacyanidoferrat (III) K₃[Fe(CN)₆] ist.

13. Verfahren nach Anspruch 1, **dadurch gekennzeichnet, dass** die Feststellung der Trübung im Infrarotbereich bei 800 - 900 nm erfolgt.

14. Verfahren nach Anspruch 13, **dadurch gekennzeichnet, dass** die Feststellung der Trübung im Infrarotbereich bei 850 nm erfolgt.
